(19) Europäisches Patentamt
European Patent Office
Office européen des brevets

(11) **EP 3 833 254 B1**

(12) **EUROPEAN PATENT SPECIFICATION**

(45) Date of publication and mention
of the grant of the patent:
**02.10.2024 Bulletin 2024/40**

(21) Application number: **19848615.1**

(22) Date of filing: **06.08.2019**

(51) International Patent Classification (IPC):
**A61M 16/12** (2006.01)   **A61B 5/0275** (2006.01)
**A61B 5/08** (2006.01)   **A61B 5/083** (2006.01)

(52) Cooperative Patent Classification (CPC):
**A61B 5/0275; A61B 5/082; A61M 16/12;**
A61B 5/0075; A61B 5/0261; A61B 5/0833;
A61B 2562/0233; A61M 2202/0208

(86) International application number:
**PCT/CA2019/051080**

(87) International publication number:
**WO 2020/028984 (13.02.2020 Gazette 2020/07)**

(54) **SYSTEM AND METHOD FOR MONITORING A BLOOD FLOW THAT DOES NOT INTERACT WITH VENTILATED LUNGS OF A PATIENT**

SYSTEM UND VERFAHREN ZUR ÜBERWACHUNG EINES BLUTFLUSSES, DER NICHT MIT VENTILIERTEN LUNGEN EINES PATIENTEN INTERAGIERT

SYSTÈME ET PROCÉDÉ DE SURVEILLANCE D'UN DÉBIT SANGUIN N'INTERAGISSANT PAS AVEC LES POUMONS VENTILÉS D'UN PATIENT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **07.08.2018 US 201862715484 P**

(43) Date of publication of application:
**16.06.2021 Bulletin 2021/24**

(73) Proprietor: **Rostrum Medical Innovations Inc.
Vancouver, British Columbia V5M 1C2 (CA)**

(72) Inventors:
• **FREDRICK, Aron
Vancouver, British Columbia V6K 3W2 (CA)**
• **GARRY, James
Burnaby, British Columbia V3N 2G4 (CA)**
• **AYOUBI, Nathan
Vancouver, British Columbia V6J 5E7 (CA)**
• **MCGREGOR, Hanna
Surrey, British Columbia V3S 8P2 (CA)**

(74) Representative: **BCF Global
Centre d'Entreprise et d'Innovation
17-21, rue Saint-Fiacre
75002 Paris (FR)**

(56) References cited:
**WO-A1-2011/143751      WO-A1-2019/028550
WO-A2-2004/073482      US-A1- 2015 272 475
US-A1- 2016 158 481      US-B1- 6 402 697**

• **PHILIP J PEYTON ET AL: "Non-invasive measurement of intrapulmonary shunt during inert gas rebreathing", PHYSIOLOGICAL MEASUREMENT, INSTITUTE OF PHYSICS PUBLISHING, BRISTOL, GB, vol. 26, no. 3, 1 June 2005 (2005-06-01), pages 309 - 316, XP020092185, ISSN: 0967-3334, DOI: 10.1088/0967-3334/26/3/014**

EP 3 833 254 B1

## Description

TECHNICAL FIELD

The present disclosure relates to the field of respiratory care. More specifically, the present disclosure relates to a system and a method for detecting and monitoring a blood flow that does not interact with ventilated lungs of a patient.

BACKGROUND

Acute Respiratory Distress Syndrome (ARDS) is a rapidly-progressing condition in which the lungs exchange gases less efficiently. This degradation of perfusion is referred to as 'pulmonary shunt', an impairment that results in more blood being 'shunted' past the lungs and reduces the uptake of oxygen and other gases to the patient's bloodstream. As a result of this shunt, some of the pulmonary blood flow cannot undergo gas-exchange with the ventilating air inhaled by the patient.

Due to pulmonary shunt, gas exchange may not take place between some of the blood flow and some of the ventilated alveoli of a patient. Thus created pulmonary deadspace in turn reflects wasted respiratory efforts in spontaneously breathing patients, or wasted respiratory assist for patients receiving mechanical ventilation.

Current methods for the detection and monitoring of ailments such as ARDS rely on invasive or time-consuming methods, such as the sampling of the patient's blood, or exposure to chest x-ray imaging. Such methods are undesirable in a variety of clinical settings. The time spent and hazard associated with such blood sampling and imaging methods are undesirable. An approach that can detect and monitor the progression of this disease in a non-invasive fashion is desired.

There is therefore a need for improved techniques for the detection and monitoring of pulmonary blood flow.

US 6,402,697 B1 describes cardiac output monitoring system having a respiratory flowmeter and a gas analyzer capable of determining cardiac output on a breath-by-breath basis by non-invasively measuring properties of respiratory gasses and applying the Fick principle. The gas analyzer has the capability to simultaneously quantify multiple gas concentrations, including inhaled and end-tidal concentrations of any constituent of respiratory gas mixtures of a known number of possible constituents, in real time on a breath-by-breath basis, by measuring independent properties of the mixture. The respiratory flowmeter determines the volumetric and mass flow rates of any gas/gasses as calculated from the product of measured total respiratory flow and the measured volumetric concentration in real time on a breath-by-breath basis. From these measurements, cardiac output can be determined on a breath-by-breath basis by applying appropriate numerical algorithms based on the Fick principal, including corrections for physiological conditions such as shunts and deadspace.

SUMMARY

The object of the invention is solved by a system according to claim 1 and a method according to claim 11. Preferred embodiments are presented in the dependent claims.

The foregoing and other features will become more apparent upon reading of the following non-restrictive description of illustrative embodiments thereof, given by way of example only with reference to the accompanying drawings.

BRIEF DESCRIPTION OF THE DRAWINGS

Embodiments of the disclosure will be described by way of example only with reference to the accompanying drawings, in which:

Figure 1 shows an idealized diagram of a human cardio-pulmonary system, illustrating an effective action of pulmonary shunt flow;

Figure 2a is a graph showing a variation over time of an arterial blood content of inert gas;

Figure 2b is a graph showing a variation over time of an end-tidal content of inert gas;

Figure 3 is a schematic diagram of a system implementing a gas control and sensing modality according to an embodiment;

Figures 4a shows a cross-sectional diagram of a gas composition sensor comprising an airway unit and a sensing unit;

Figure 4b shows a light path within the gas composition sensor of Figure 4a;

Figure 5a shows an alternative cross-sectional diagram of the gas composition sensor, showing how various parts may be remote or proximate to the gas composition sensor.

Figure 5b shows a light path within the gas composition sensor of Figure 5a;

Figure 6a shows a cross-sectional side view of a gas composition sensor comprising an airway unit and a sensing unit according to another embodiment;

Figure 6b is a cross-sectional front view of the gas composition sensor of Figure 6a;

Figure 6c is a transparent, perspective view of the gas composition sensor of Figure 6a; and

Figure 7 is a sequence diagram showing operations of a method for monitoring a blood flow that does not interact with ventilated lungs of a patient.

DETAILED DESCRIPTION

Various aspects of the present disclosure generally address one or more of the problems associated with the detection and monitoring of the pulmonary blood flow, in particular when some the pulmonary blood flow cannot undergo gas-exchange with the ventilating air inhaled by the patient.

Generally speaking, the present disclosure introduces a method and a system for the determination of impaired gas exchange in the lungs of human patients. The disclosed technology allows a determination of trends in pulmonary shunt through gas uptake, excretion, and recirculation.

In one aspect, a system is designed to be used on patients who are mechanically-ventilated. The system may also be used on spontaneously breathing patients. The system provides information about the efficiency of the lung as an organ of gas-exchange and, in particular but without limitation, addresses the progression of ailments such as Acute Respiratory Distress Syndrome (ARDS).

In another aspect, the disclosed system provides a patient with controlled volumes of inhaled gases, and monitors the concentration of those gases in the inhaled and exhaled breath of the patient. Sensitive measurement of those gases gives rise to a mechanism for deducing the perfusion characteristics of the lung, allowing a measure of how much blood flow does not take part in gas exchange.

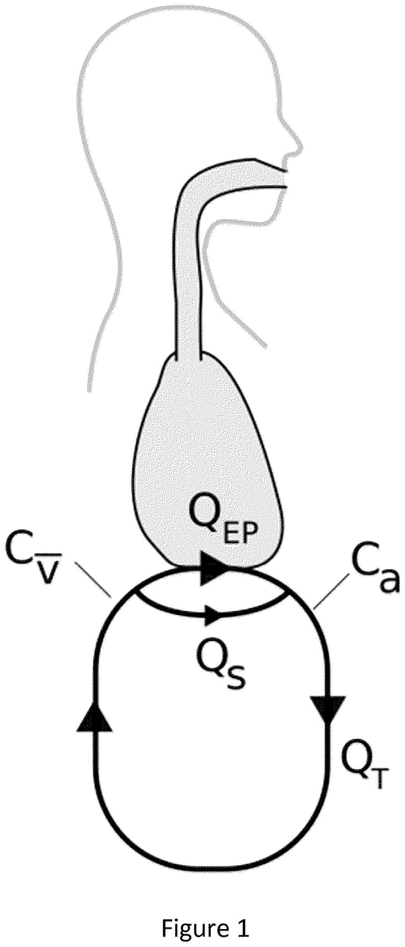

Figure 1 shows an idealized diagram of a human cardio-pulmonary system, illustrating an effective action of pulmonary shunt flow. Let the patient be ventilated with a gas, with a certain solubility, at an inhaled fraction $F_1$, and in a tidal volume $V_T$, with a functional reserve capacity (FRC), $V_{FRC}$. The alveolar space has a volume that is defined as the sum of the tidal volume and the functional residual capacity. At the end of exhalation, the alveolar space has an average make up, $F_E$, which is mirrored in the end-tidal composition of the exhale. A mass-balance of two breaths is considered, where the inhaled gaseous agent is able to undergo gas exchange with the pulmonary blood flow for a duration, t, which scales with the duration of the inhalation. With uniform inhaled content, changes in the end-tidal values of successive breaths, $\Delta F_E$, arise from two sources: a change in the gas content makeup of the FRC, or gas-exchange with the effective pulmonary blood flow, $Q_{EP}$. For a blood-soluble gas described in this disclosure, these two terms are coupled in equation [1]:

$$[C_a(t) - C_{\bar{v}}(t)]Q_{EP} \cdot t + \Delta F_E \cdot V_{FRC} = V_T(F_I - F_E) \qquad [1]$$

If the mixed venous concentration, $C_{\bar{v}}$, is constant, or in the case of an inert (non-metabolic) and non-toxic gas, absent, a number of methods exist that allow the values of the effective pulmonary non-shunted blood flow, $Q_{EP}$, to be found with the use of mixtures of inhaled gases that have particularly low and high solubility values. Such single-breath, rebreathing, and perturbation methods are known in the field and will not be described further.

There are no currently known non-invasive methods that reveal the shunted blood flow $Q_S$. The shunted flow and the effective pulmonary blood flow, $Q_{EP}$, are equal to the cardiac output, $Q_T$, which is the total blood flow that circulates inhaled gases around the systemic vasculature, and back to the lungs. The mixed venous blood therefore has a 'memory' of earlier contents of the FRC and acts like a lossy delay line in response to stimuli arising from respiration.

The present disclosure introduces two stimulus models by which the patient's ventilation may be modified and monitored.

In a first stimulus model, a short series of breaths (for example less than five breaths) enriched with a gas having a substantial solubility in blood are provided to the patient. For example, nitrous oxide, with its Ostwald coefficient of 0.47, is a good nominal candidate in the present disclosure. The gas is selected based on its ability to readily diffuse through the alveolar membranes. In most cases, the selected gas is composed of relatively small molecules. The effective pulmonary blood flow's content is influenced by this stimulus with a characteristic timescale that varies as the ratio of $V_{FRC}/V_t$, acting as a low-pass filter to the ventilation-borne stimulus. The blood that enters the systemic circulation is a blend of the 'shunted' flow that does not undergo gas-exchange, Qs, and that which has experienced gas exchange in the lung.

The action of recirculation in this first stimulus model is that of changing the mixed venous blood content presented to the lungs, $C_{\bar{v}}$. If, prior to that moment, the FRC has a static makeup, this change in blood content leads to a change in the end-tidal gas content. The magnitude of the change reflects inversely the pulmonary shunt fraction, $f_S = O_S/Q$.

In a second stimulus model, a longer series of breaths enriched with a gas having a substantial solubility in blood is provided to the patient. Such gas-enriched breathing persists for a time period longer than the average recirculation time of the patient's systemic vasculature. In this second stimulus model, the end-tidal makeup of exhaled breaths is noted. Upon recirculation of the gas-enriched blood, the first term in equation [1] changes, reducing the affinity of the effective pulmonary blood for the inhaled blood-soluble gas. This degree of uptake reflects inversely the pulmonary shunt fraction, $f_S$. A scenario in which the series of breaths is selected so that it has a duration that is substantially equat to the average recirculation time of the patient's systemic vasculature is also contemplated.

It is assumed throughout this disclosure that the transport of the gas-enriched blood through the body's vasculature and organs may be treated as a process that changes by a negligible amount, if at all, over the timescale associated with the stimuli supplied to the patient via their breathing. Without loss of generality, the end-tidal exhaled breath content may be related to the earlier concentration history of the soluble gas in the mixed-venous blood entering the lung at the pulmonary artery, $C_{\bar{v}}(t)$.

The link between the mixed venous blood content and the prior history of inhaled gas content is treated as a transfer function. Plausible functions that satisfy this criterion are delayed spectral functions, such as low-pass filters, that delay and attenuate signals arising in the arterial composition. Equation [2] applies for some function $X$ that describes the dispersion in a given patient, arising from a bolus of gas dissolved into the systemic vasculature:

$$C_{\bar{v}}(t) = f(C, t, X) \qquad [2]$$

In which $C$ is a generalized concentration of the blood-soluble physiologically inert gas in the body over time $t$.

Upon its return to the lung, the blood that is shunted 'past' the lung does not differ from that which is presented to the ventilated alveoli of the lung. The dissolved agent is chosen such that it readily diffuses across the alveolar membrane and so the partial pressure of the blood that perfuses the ventilated alveoli is in equilibrium with the gas in those alveoli.

Operating the system according to the second stimulus model, the end-tidal exhaled composition is used to monitor the relationship that joins the end-capillary partial pressures to the end-tidal qualities. This relationship is compounded from the transfer function in equation [2], $f$, which is presumed to be invariant over the duration of the applied challenge, and the connection between the end-capillary content and the arterial content, wherein $q$ is unknown, but is expected to change if the patient's severity of ARDS alters.

In both cases, whether through the uptake to the systemic blood during a series of gas-rich breaths, or the elimination from the systemic blood after a series of gas-rich breaths, changes in the end-tidal concentration provide a measure of the degree of pulmonary shunt. This causal relationship relies, firstly, upon the end-tidal gas content being an accurate reflection of the alveolar airspace. Responsive sensors may be used to detect that portion of the exhale. The second support for this relationship arises from a link between the time-series of the mixed-venous blood and the make-up of the alveolar airspace.

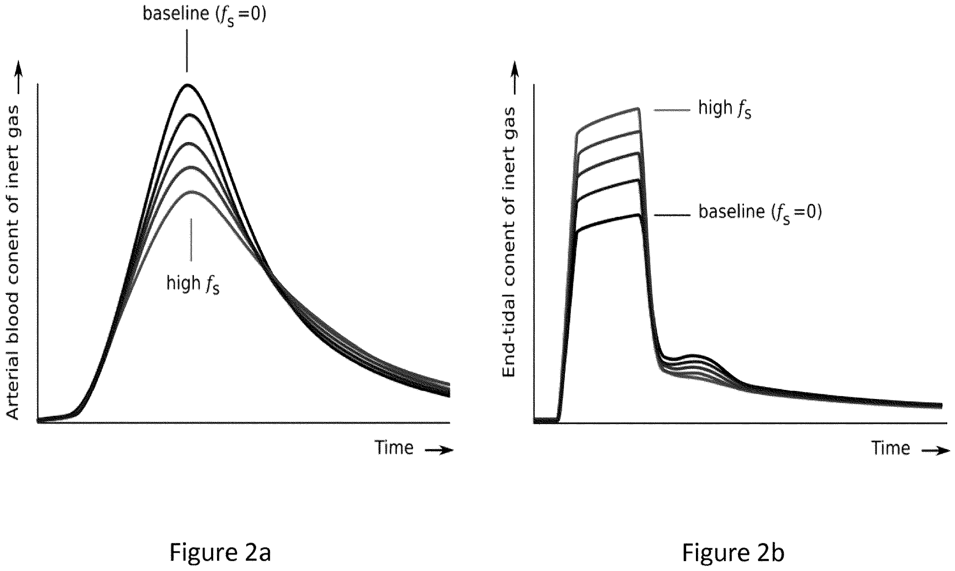

Figure 2a is a graph showing a variation over time of an arterial blood content of inert gas. Figure 2b is a graph showing a variation over time of an end-tidal content of inert gas. Figures 2a and 2b are based on a detailed physiological model of an adult pig, devised to explore these relations under various stimulus scenarios. The graphs in Figures 2a and 2b show a model-derived response of a pig's arterial blood and exhalations to a number of breaths which contain an abiogenic inert gas, in the present case nitrous oxide ($N_2O$). This model predicts the effect of an increasing pulmonary shunt fraction on the arterial and end-tidal makeup of a patient being ventilated for a number of breaths with a gas that is biologically inert but blood-soluble.

In the absence of pulmonary shunt (baseline, $f_s=0$), the gas is readily uptaken to the systemic blood as revealed by a lowered content in the exhale during those breaths. In summary, the graphs in Figures 2a and 2b illustrate how the presence of a substantial shunt fraction, or high *fs,* leads to changes in the amount of gas that is uptaken to the blood during the few breaths of the stimulus, and also alters the post-stimulus elimination of the blood-soluble gas.

Specifically, Figures 2a and 2b show that:

a) a raised shunt fraction diminishes the reappearance of the gas in the alveolar space upon the first recirculation of the systemic blood flow; and

b) a raised shunt fraction leads to the slower elimination of the gas from the body, evinced by the lowered end-tidal gas content for that inert gas long after the cessation of the stimulus. A steady respiratory state of the patient is reached when the end-tidal concentration of the inert gas becomes stable.

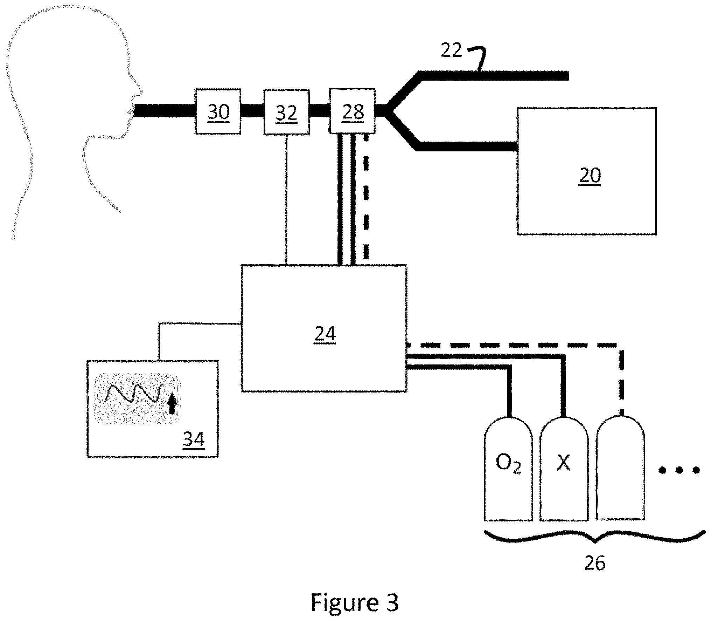

Figure 3 is a schematic diagram of a system implementing a gas control and sensing modality according to an embodiment. The system is an evolution of co-owned International Application no. PCT/CA2018/050957, entitled "Method And System For Estimating The Efficiency Of The Lungs Of A Patient", filed on August 6, 2018. A mechanical ventilator 20 delivers a life-sustaining airflow to the patient through a conventional airway circuit 22. A control unit 24 arranges for a number of breaths to be inhaled that have specific concentrations of gases from gas sources, such as tanks 26. These gases are mixed in a gas mixer 28, and a resulting blended flow passes through a heat and moisture exchange device 30. The control unit 24 is configured to ensure that the concentration of oxygen in the inhaled breaths is acceptable from the perspective of clinical care. A makeup of the blended flow is noted by a gas composition sensor 32. The method embodied in the control unit 24 performs an analysis, the results of which may be presented to the operator via a display unit 34 that may be contained within the control unit 24. Alternatively, the display unit 34 may be adjacent to or distant from the control unit 24, being connected electronically or via a wireless connection.

More than one tank 26 is shown in Figure 3 because the addition of a non-metabolic gas dilutes inspired air and may lead to hypoxia. Therefore, in an embodiment, one of the tanks 26 may hold oxygen. One of the other gases, denoted 'X' on Figure 3, may be chosen to have a desired solubility in human blood. These gases are sensed by the gas composition sensor 32 in such a way that the concentration profiles of these gases throughout the patient's respiration can be measured. One or more of those soluble gases may have no biogenic origin within the patient and thus play no role in the patient's metabolism, such a gas being referred to as being physiologically inert. The gas composition sensor 32 may employ non-dispersive infra-red spectroscopy to deduce the intra-breath gas concentrations.

The system of Figure 3 may be used for monitoring a blood flow that does not interact with the ventilated lungs of a patient, for example when the patient has a shunted pulmonary blood flow. In the system, the mechanical ventilator 20 is a first source of a respiratory gas. One of the tanks 26 is a second source containing a blood-soluble metabolically inert gas. The gas mixer 28, which is connected to the first and second sources, delivers a blend of the respiratory gas and of the blood-soluble metabolically inert gas to an airway of the patient. The gas composition sensor 32 measures a fraction of the blood-soluble metabolically inert gas present in inhaled and exhaled breaths in the airway of the patient. The control unit 24 performs the following operations:

a) causes the gas mixer 28 to deliver a first number of breaths to the patient, the breaths of the first number of breaths containing the respiratory gas and an amount of the blood-soluble metabolically inert gas;

b) determines, based on measurements from the gas composition sensor 32, an end-tidal concentration value of the blood-soluble metabolically inert gas in exhaled breaths of the patient during the delivery of the first number of breaths to the patient;

c) repeats b) until at least one of the following occurs: i) a predetermined time duration after a) elapses, or ii) at least two successive end-tidal concentration values of the blood-soluble metabolically inert gas in exhaled breaths of the patient are substantially equal;

d) after c), causes the gas mixer 28 to deliver a second number of breaths to the patient, the breaths of the second number of breaths containing none of the blood-soluble metabolically inert gas; and

e) determines, based on measurements from the gas composition sensor 32, an end-tidal concentration value of the blood-soluble metabolically inert gas in exhaled breaths of the patient during the delivery of the second number of breaths to the patient.

The control unit 24 estimates a relative fraction of the blood flow that does not interact with the ventilated lungs of the patient based on variations of the end-tidal concentration values in the first number of breaths, or in the second number of breaths or in the first and second number of breaths. The control unit 24 may also cause the gas mixer 28 to inject oxygen in any breath delivered to the patient when an oxygen proportion in the breaths delivered to the patient and measured by the gas composition sensor 32 is under a predetermined threshold. The control unit 24 may further set the first number of breaths so their combined duration is less than, or more than, an average time of blood recirculation in lungs of the patient, depending on whether the first or second operating mode is selected. In the second operating mode, the control unit 24 may also control a delivery of the first number of breaths continues until measurements from the gas composition sensor 32 shows a stable end-tidal concentration value of the blood-soluble metabolically inert gas in exhaled breaths of the patient. Various combinations of these features may be implemented in various embodiments of the system.

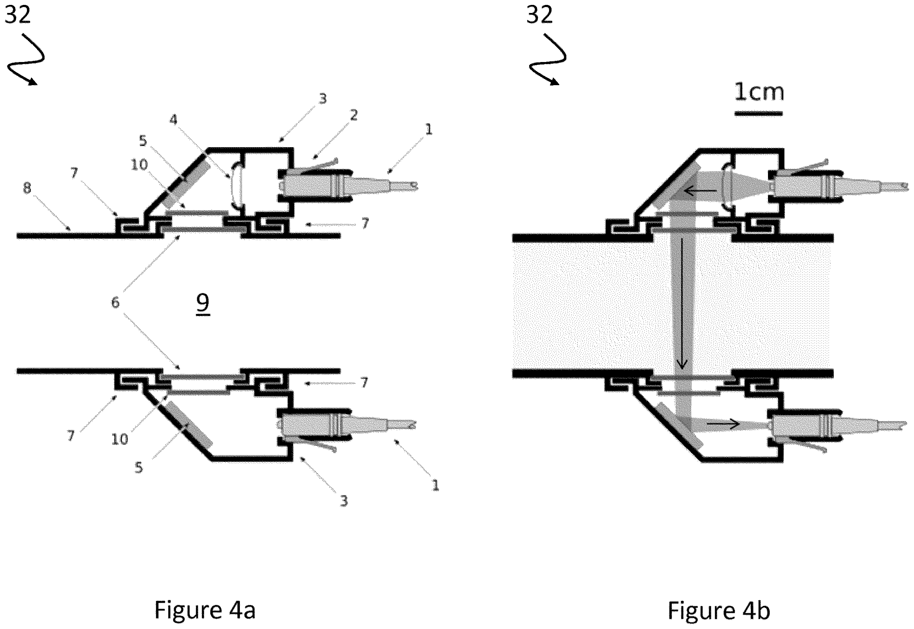

Figures 4a shows a cross-sectional diagram of a gas composition sensor comprising an airway unit and a sensing unit. Figure 4b shows a light path within the gas composition sensor of Figure 4a. Figures 4a and 4b are not to scale. Dimensions shown on Figure 4b are for illustration purposes and do not limit the present disclosure. On Figure 4b, a direction of the light path is indicated by use of arrows. The gas composition sensor 32 of Figure 4a and 4b is one possible embodiment of the gas composition sensor 32 introduced in the description of Figure 3. In reference to Figure 4a, a respiratory air-stream 9 passes to and from a patient through an airway housing part 8. A sensing part 3 fits over the airway housing part 8 through the use of physical slides and latches 7. The sensing part 3 carries a pair of thin windows 6 that are substantially transparent to infra-red light, for example having a wavelength in the region of about 4.0 through 4.7 micron. Other wavelength ranges may be contemplated depending on the actual type of blood-soluble metabolically inert gas being used. A light-guiding conduit, for example a fiber optic 1, carries infra-red radiation to a set of optics that may comprise one or more refractive elements, for example a lens 4, and at least one reflective element, for example a mirror 5. The infra-red radiation passes through a window 10 that is captive to the sensing part 3. After passing through another window 6, which is captive to the airway housing part 8, the light passes through the respiratory air-stream 9 and the infra-red radiation traverses another pair of windows, further optics, and illuminates a second light-guiding conduit, for example another fiber optic 1.

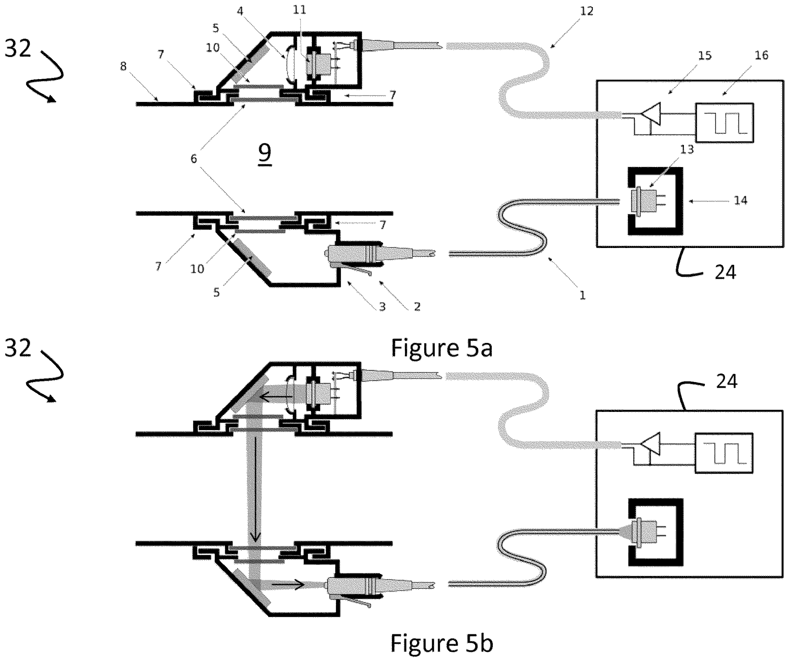

An alternative embodiment, using the same sensing modality is also contemplated, in which one or both of the light source and the sensor are mounted in the airway housing part 8 and connected electronically to the control unit 24 (Figure 3). An example of this alternative embodiment is shown in Figure 5a, which shows an alternative cross-sectional diagram of the gas composition sensor, showing how various parts may be remote or proximate to the gas composition sensor, with Figure 5b showing a light path within the gas composition sensor of Figure 5a. Figures 5a and 5b are not to scale. In Figure 5a, the infra-red detector is retained in the control unit 24. The infra-red source 11 is located in the gas composition sensor 32 on the respiratory air-stream 9, being powered electrically via a flexible cable 12 arising from the control unit 24. A waveform generator 16 and an amplifier 15 are connected via a cable 12 to the infra-red source 11. In an embodiment, the infra-red sensor 13 is located in an isothermal housing 14 and is therefore sheltered from temperature variations, physical shocks, and mechanical damage. The infra-red sensor 13 may include a number of optical filters (not shown) that allow a number of specific wavelength ranges of light to be sensed.

In this alternative embodiment, the infra-red detector 13 remains in the control unit 24 where it can be held in an isothermal and vibration-shielded state. In such an embodiment, the infra-red source 11 is present in the part that fits around the airway part. Figure 5b highlights the light path, indicated by use of arrows, within the embodiment of Figure 5a.

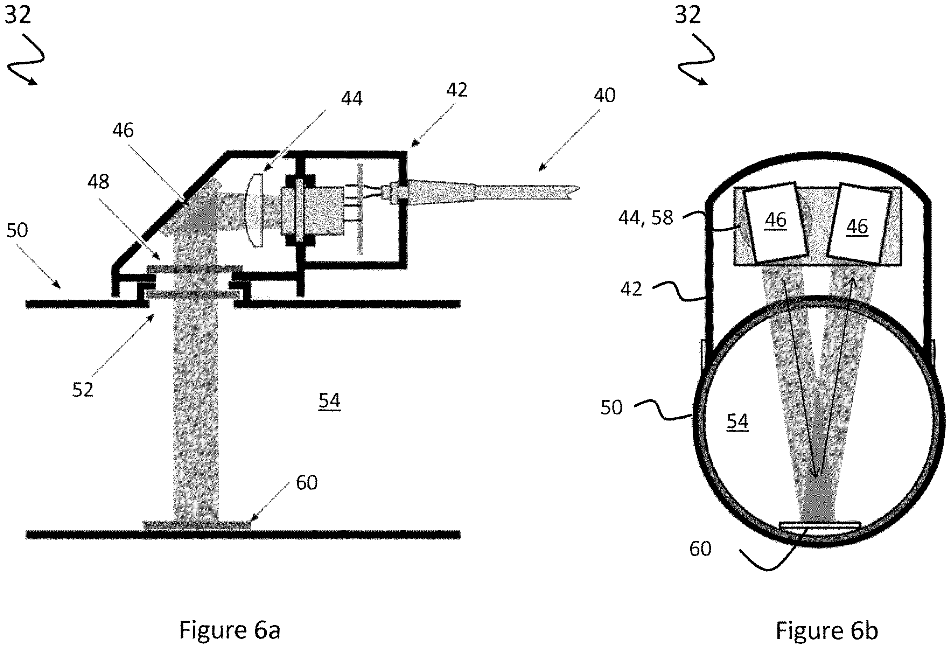

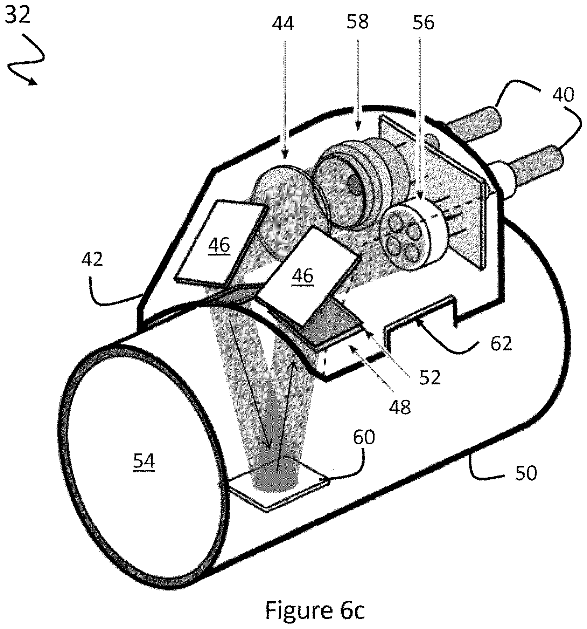

Figure 6a shows a cross-sectional side view of a gas composition sensor comprising an airway unit and a sensing unit according to another embodiment. Figure 6b is a cross-sectional front view of the gas composition sensor of Figure 6a. Figure 6c is a transparent, perspective view of the gas composition sensor of Figure 6a. Figures 6a, 6b and 6c are not to scale. In the embodiment of Figures 6a, 6b and 6c, the gas composition sensor 32 comprises a sensor housing 42 that holds active optical components, as well as an airway housing 50 through which flow a respiratory air-stream 54. A latching arrangement 62 attaches the sensor housing 42 and the airway housing 50. Electric wires 40 are connected to a collimated infra-red source 58 and to an infra-red sensor 56, which are both located within the sensor housing 42. The sensor housing 42 also contains a pair of mirrors 46 placed at an angle in front of the infra-red source 58 and of the infra-red sensor 56 for directing light to and from windows 48 and 52. A lens 44 allows focusing the light from the infra-red source 58 toward a first mirror 46. The windows 48 and 52 allow light to traverse between the sensor housing 42 and the airway housing 50.

On Figures 6b and 6c, a direction of a light path is indicated by use of arrows. The light emitted by the infra-red source 58 and deflected by a first one of the mirrors 46 traverses the windows 48 and 52 and traverses the entire width of the airway housing 50 before reaching a mirror 60. The light is then reflected by the mirror 60 in a substantially opposed direction toward the windows 52 and 48 and reaches a second one of the mirrors 46, being deflected toward the infra-red sensor 56. The configuration of Figures 6a, 6b and 6c, which includes the mirror 60, increases a length of the light path between the infra-red source 58 and the infra-red sensor 56. A large portion of this extended light path actually traverses the airway housing 50 twice, the light being thus exposed to the respiratory air-stream 54 twice. Such a configuration allows the beam of infra-red light to double-back on itself, doubling the path length taken by the light through the airway housing 50 when compared to the embodiments of Figures 4a and 5a. For a given infra-red sensor and infra-red source, this longer path length supports a lower minimum-detectable concentration of various gases present in the airway housing 50. Use of multiple reflective elements at multiple positions within the airway housing 50 allowing to further increase the path length is also contemplated.

Regardless of the actual construction of the gas composition sensor 32, with a suitable set of optical windows in the infra-red sensor 13, is able to rapidly measure concentration of the gases in the respired air from the patient, these measurements being obtained in less than a duration of an inspiratory or expiratory phase of the patient. In a particular feature of the present ventilation technology, the uptake to the patient's blood of a blood-soluble physiologically inert gas occurs at a rate that is influenced by the flow-rate of the non-shunted pulmonary blood flow. By monitoring the rate at which such inert gases are abstracted into the patient's blood flow in response to changes in the delivered gas content, a number of cardio-pulmonary qualities can be deduced, as expressed in the above-mentioned International Application no. PCT/CA2018/050957. In another particular feature of the present ventilation technology, the rate at which that gas is eliminated from a patient following the uptake of such a physiologically inert gas is influenced by the relative magnitude of the shunted pulmonary blood flow with respect to the cardiac output, the elimination rate being slowed in scenarios with raised shunted pulmonary blood flow.

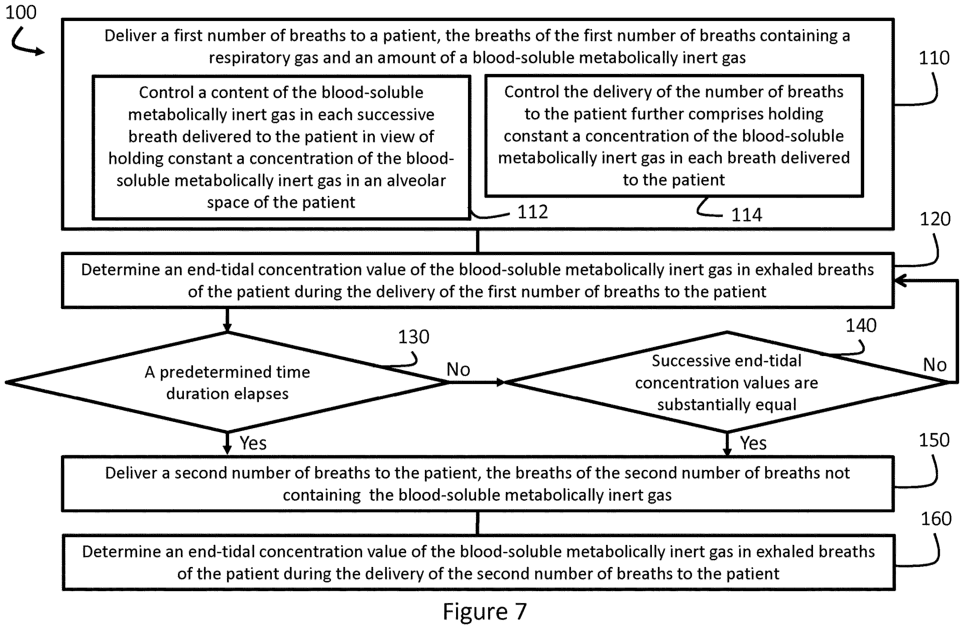

Figure 7 is a sequence diagram showing operations of a method for monitoring a blood flow that does not interact with ventilated lungs of a patient. On Figure 7, a sequence 100 comprises a plurality of operations, some of which may be executed in variable order, some of the operations possibly being executed concurrently, some of the operations being optional. The sequence 100 starts with operation 110, in which a first number of breaths is delivered to a patient. The breaths of the first number of breaths contain a respiratory gas and an amount of a blood-soluble metabolically inert gas. Operation 110 may optionally comprise one of sub-operations 112 or 114. In sub-operation 112, a content of the blood-soluble metabolically inert gas in each successive breath delivered to the patient is controlled in view of holding constant a concentration of the blood-soluble metabolically inert gas in an alveolar space of the patient. Alternatively, in sub-operation 114, control of the delivery of the number of breaths to the patient is made by holding constant a concentration of the blood-soluble metabolically inert gas in each breath delivered to the patient. Other manners of controlling the content of the blood-soluble metabolically inert gas in each successive breath delivered to the patient are also contemplated so the examples of sub-operations 112 and 114 are not intended to limit the present disclosure.

At operation 120, an end-tidal concentration value of the blood-soluble metabolically inert gas in exhaled breaths of the patient is determined while the delivery of the first number of breaths to the patient is ongoing. Otherwise stated, operation 120 may be concurrent with operation 110. The end-tidal concentration value of the blood-soluble metabolically inert gas may be determined once per expiratory phase of the patient, at a faster rate, or at a slower rate.

Operation 130 verifies if a predetermined time duration has elapsed since the beginning of operation 110. If so, the sequence 100 continues at operation 150. If not, the sequence 100 continues at operation 140 in which it is determined if at least two successive end-tidal concentration values of the blood-soluble metabolically inert gas in exhaled breaths of the patient are substantially equal. If so, the sequence 100 continues at operation 150. If not, the sequence 100 returns to operation 120. If operation 140 continues having negative results in successive cycles of the sequence 100, operation 130 will eventually determine that the predetermined duration has elapsed. Consequently, operation 150 will eventually be reached.

At operation 150, a second number of breaths is delivered to the patient. In this operation, the breaths of the second number of breaths do not contain the blood-soluble metabolically inert gas. In the context of the present disclosure, trace amounts of the blood-soluble metabolically inert gas might remain in the breaths of the second number of breaths, especially if the gas mixer 28 does not have perfect isolation characteristics. The skilled reader will readily appreciate that the intent of operation 150 is that no clinically significant amount of the blood-soluble metabolically inert gas remains in these breaths.

At operation 160, an end-tidal concentration value of the blood-soluble metabolically inert gas in exhaled breaths of the patient is determined during the delivery of the second number of breaths to the patient. Operations 150 and 160 may be performed concurrently.

Variations of the end-tidal concentration values determined at operations 120 and/or 160 are used to estimate a relative fraction of the blood flow that does not interact with the ventilated lungs of the patient.

Those of ordinary skill in the art will realize that the description of the system and the method for monitoring a blood flow that does not interact with ventilated lungs of a patient are illustrative only and are not intended to be in any way limiting. Other embodiments will readily suggest themselves to such persons with ordinary skill in the art having the benefit of the present disclosure. Furthermore, the disclosed system and method may be customized to offer valuable solutions to existing needs and problems related to the detection and monitoring of pulmonary blood flow. In the interest of clarity, not all of the routine features of the implementations of the system and method are shown and described. In particular, combinations of features are not limited to those presented in the foregoing description as combinations of elements listed in the appended claims form an integral part of the present disclosure. It will, of course, be appreciated that in the development of any such actual implementation of the system and method numerous implementation-specific decisions may need to be made in order to achieve the developer's specific goals, such as compliance with application-related, system-related, and business-related constraints, and that these specific goals will vary from one implementation to another and from one developer to another. Moreover, it will be appreciated that a development effort might be complex and time-consuming, but would nevertheless be a routine undertaking of engineering for those of ordinary skill in the field of respiratory care having the benefit of the present disclosure.

In accordance with the present disclosure, the control unit 24 described herein may be implemented using various types of operating systems, computing platforms, network devices, computer programs, and/or general purpose machines. In addition, those of ordinary skill in the art will recognize that devices of a less general purpose nature, such as hardwired devices, field programmable gate arrays (FPGAs), application specific integrated circuits (ASICs), or the like, may also be used. Where a method comprising a series of operations is implemented by the control unit 24, a processor operatively connected to a memory, or a machine, those operations may be stored as a series of instructions readable by the machine, processor or computer, and may be stored on a non-transitory, tangible medium.

The present disclosure has been described in the foregoing specification by means of non-restrictive illustrative embodiments provided as examples. These illustrative embodiments may be modified at will. The scope of the invention is defined in the appended claims.

## Claims

1.  A system for monitoring a blood flow that does not interact with ventilated lungs of a patient, comprising:

    a first source (20) of a respiratory gas;
    a second source (26) containing a blood-soluble metabolically inert gas;
    a gas mixer (28) connected to the first and second sources and adapted to deliver a blend of the respiratory gas and of the blood-soluble metabolically inert gas to an airway of the patient;
    a gas composition sensor (32) adapted to measure a concentration of the blood-soluble metabolically inert gas present in inhaled and exhaled breaths in the airway of the patient; and
    a control unit (24) operatively connected to the gas mixer (28) and to the gas composition sensor (32), wherein the control unit (24) is adapted to:

        a) cause the gas mixer (28) to deliver a first number of breaths to the patient, the breaths of the first number of breaths containing the respiratory gas and an amount of the blood-soluble metabolically inert gas, each breath of the first number of breaths containing a clinically acceptable concentration of oxygen;
        b) determine, based on measurements from the gas composition sensor (32), an end-tidal concentration value of the blood-soluble metabolically inert gas in exhaled breaths of the patient during the delivery of the first number of breaths to the patient;
        c) repeat b) until at least one of the following occurs: *i*) a predetermined time duration after a) elapses, or *ii*) at least two successive end-tidal concentration values of the blood-soluble metabolically inert gas in exhaled breaths of the patient are substantially equal;
        d) after c), cause the gas mixer (28) to deliver a second number of breaths to the patient, the breaths of the second number of breaths containing none of the blood-soluble metabolically inert gas;
        e) determine, based on measurements from the gas composition sensor (32), an end-tidal concentration value of the blood-soluble metabolically inert gas in exhaled breaths of the patient during the delivery of the second number of breaths to the patient; and
        f) estimate a relative fraction of the blood flow that does not interact with the ventilated lungs of the patient based on variations of the end-tidal concentration values in the first number of breaths, or in the second number of breaths or in the first and second number of breaths.

2.  The system of claim 1, further comprising an airway circuit (22) adapted for being connected to the airway of the patient.

3.  The system of claim 1 or 2, wherein the first source (20) of the respiratory gas is a mechanical ventilator (20).

4.  The system of any one of claims 1 to 3, further comprising a source of oxygen (26) operatively connected to the gas mixer (28), wherein the control unit (24) is further adapted to cause the gas mixer (28) to inject oxygen in any breath delivered to the patient when an oxygen proportion in the breaths delivered to the patient and measured by the gas composition sensor (32) is under a predetermined threshold.

5.  The system of any one of claims 1 to 4, wherein the blood-soluble metabolically inert gas is nitrous oxide.

6. The system of any one of claims 1 to 5, wherein in a first operating mode, the control unit (24) sets the first number of breaths so their combined duration is less than an average time of blood recirculation in lungs of the patient.

7. The system of any one of claims 1 to 6, wherein in a second operating mode, the control unit (24) sets the first number of breaths so that their duration exceeds the average time of blood recirculation in the lungs of the patient.

8. The system of claim 7, wherein a delivery of the first number of breaths continues until measurements from the gas composition sensor (32) show a stable end-tidal concentration value of the blood-soluble metabolically inert gas in exhaled breaths of the patient.

9. The system of any one of claim 1 to 8, wherein the gas composition sensor (32) comprises:

   a first part including a set of optical elements (4, 5, 6, 10, 44, 46, 48, 52, 60) adapted to allow infra-red light to be shone across the airway of the patient; and
   a second part comprising an infra-red source (11, 58) and an infra-red detector (13, 56), the second part being disjoint from the airway of the patient and being connected to the set of optical elements of the first part by an optical conduit.

10. The system of claim 9, wherein the infra-red source (11, 58) and the infra-red detector (13, 56) are positioned proximate to the airway of the patient, the gas composition sensor (32) further comprising a reflective element (5, 46, 60) positioned within an optical path defined between the infra-red source (11, 58) and the infra-red detector (13, 56).

11. A method (100) for monitoring a blood flow that does not interact with ventilated lungs of a patient with a system according to any one of claims 1 to 10, wherein the method comprises:

   a) delivering (110) a first number of breaths to the patient, the breaths of the first number of breaths containing a respiratory gas and an amount of a blood-soluble metabolically inert gas, each breath of the first number of breaths containing a clinically acceptable concentration of oxygen;
   b) determining (120) an end-tidal concentration value of the blood-soluble metabolically inert gas in exhaled breaths of the patient during the delivery of the first number of breaths to the patient;
   c) repeating b) until at least one of the following occurs: *i*) a predetermined time duration after a) elapses, or *ii*) at least two successive end-tidal concentration values of the blood-soluble metabolically inert gas in exhaled breaths of the patient are substantially equal (140);
   d) after c), delivering (150) a second number of breaths to the patient, the breaths of the second number of breaths containing none of the blood-soluble metabolically inert gas;
   e) determining (160) an end-tidal concentration value of the blood-soluble metabolically inert gas in exhaled breaths of the patient during the delivery of the second number of breaths to the patient; and
   f) estimating a relative fraction of the blood flow that does not interact with the ventilated lungs of the patient based on variations of the end-tidal concentration values in the first number of breaths, or in the second number of breaths or in the first and second number of breaths.

12. The method of claim 11, wherein delivering (110) the first number of breaths to the patient further comprises controlling (112) a content of the blood-soluble metabolically inert gas in each successive breath delivered to the patient in view of holding constant a concentration of the blood-soluble metabolically inert gas in an alveolar space of the patient.

13. The method of claim 11, wherein delivering (110) the first number of breaths to the patient further comprises holding constant (114) a concentration of the blood-soluble metabolically inert gas in each breath delivered to the patient.

**Patentansprüche**

1. System zum Überwachen eines Blutflusses, der nicht mit der beatmeten Lunge eines Patienten interagiert, umfassend:

   eine erste Quelle (20) eines Atemgases;
   eine zweite Quelle (26), die ein blutlösliches, metabolisch inertes Gas enthält;
   einen Gasmischer (28), der mit der ersten und der zweiten Quelle verbunden ist und eingerichtet ist, eine Mischung des Atemgases und des blutlöslichen metabolisch inerten Gases an einen Atemweg des Patienten zu abzugeben;

einen Gaszusammensetzungssensor (32), der zum Messen einer Konzentration des in den Ein- und Ausatemzügen in den Atemwegen des Patienten vorhandenen blutlöslichen metabolisch inerten Gases ausgelegt ist; und eine Steuereinheit (24), die mit dem Gasmischer (28) und dem Gaszusammensetzungssensor (32) in Wirkverbindung steht,

wobei die Steuereinheit (24) zu Folgendem ausgelegt ist:

a) Veranlassen, dass der Gasmischer (28) eine erste Anzahl von Atemzügen an den Patienten abgibt, wobei die Atemzüge der ersten Anzahl von Atemzügen das Atemgas und eine Menge des blutlöslichen metabolisch inerten Gases enthalten, wobei jeder Atemzug der ersten Anzahl von Atemzügen eine klinisch akzeptable Konzentration an Sauerstoff enthält;

b) Bestimmen eines endexspiratorischen Konzentrationswerts des blutlöslichen metabolisch inerten Gases in den Ausatemzügen des Patienten während der Abgabe der ersten Anzahl von Atemzügen an den Patienten, basierend auf Messungen des Gaszusammensetzungssensors (32);

c) Wiederholen von b), bis mindestens eines der folgenden eintritt: i) eine vorbestimmte Zeitdauer nach a) verstreicht, oder ii) mindestens zwei aufeinander folgende endexspiratorische Konzentrationswerte des blutlöslichen metabolisch inerten Gases in den Ausatemzügen des Patienten im Wesentlichen gleich sind;

d) nach c) Veranlassen, dass der Gasmischer (28) eine zweite Anzahl von Atemzügen an den Patienten abgibt, wobei die Atemzüge der zweiten Anzahl von Atemzügen kein blutlösliches metabolisch inertes Gas enthalten;

e) Bestimmen eines endexspiratorischen Konzentrationswerts des blutlöslichen metabolisch inerten Gases in den Ausatemzügen des Patienten während der Abgabe der zweiten Anzahl von Atemzügen an den Patienten, basierend auf Messungen des Gaszusammensetzungssensors (32); und

f) Schätzen eines relativen Anteils des Blutflusses, der nicht mit der beatmeten Lunge des Patienten interagiert, basierend auf Schwankungen der endexspiratorischen Konzentrationswerte in der ersten Anzahl von Atemzügen oder in der zweiten Anzahl von Atemzügen oder in der ersten und zweiten Anzahl von Atemzügen.

2. System nach Anspruch 1, ferner umfassend einen Atemwegskreislauf (22), der dazu ausgelegt ist, mit dem Atemweg des Patienten verbunden zu werden.

3. System nach Anspruch 1 oder 2, wobei die erste Quelle (20) des Atemgases ein mechanisches Beatmungsgerät (20) ist.

4. System nach einem der Ansprüche 1 bis 3, ferner umfassend eine Sauerstoffquelle (26), die mit dem Gasmischer (28) in Wirkverbindung steht, wobei die Steuereinheit (24) ferner dazu ausgelegt ist, den Gasmischer (28) zu veranlassen, Sauerstoff in jeden an den Patienten abgegebenen Atemzug zu injizieren, wenn ein Sauerstoffanteil in den an den Patienten abgegebenen Atemzügen, der durch den Gaszusammensetzungssensor (32) gemessen wird, unter einem vorbestimmten Schwellenwert liegt.

5. System nach einem der Ansprüche 1 bis 4, wobei das blutlösliche metabolisch inerte Gas Distickstoffoxid ist.

6. System nach einem der Ansprüche 1 bis 5, wobei die Steuereinheit (24) in einer ersten Betriebsart die erste Anzahl von Atemzügen so einstellt, dass deren kombinierte Dauer kleiner als eine durchschnittliche Zeit der Blutrezirkulation in der Lunge des Patienten ist.

7. System nach einem der Ansprüche 1 bis 6, wobei die Steuereinheit (24) in einer zweiten Betriebsart die erste Anzahl von Atemzügen so einstellt, dass deren Dauer die durchschnittliche Zeit der Blutrezirkulation in der Lunge des Patienten übersteigt.

8. System nach Anspruch 7, wobei eine Abgabe der ersten Anzahl von Atemzügen fortgesetzt wird, bis Messungen von dem Gaszusammensetzungssensor (32) einen stabilen endexspiratorischen Konzentrationswert des blutlöslichen metabolisch inerten Gases in Ausatemzügen des Patienten zeigen.

9. System nach einem der Ansprüche 1 bis 8, wobei der Gaszusammensetzungssensor (32) Folgendes umfasst:

einen ersten Teil, der einen Satz von optischen Elementen (4, 5, 6, 10, 44, 46, 48, 52, 60) umfasst, die dazu ausgelegt sind, Infrarotlicht über den Atemweg des Patienten strahlen lassen; und
einen zweiten Teil, der eine Infrarotquelle (11, 58) und einen Infrarotdetektor (13, 56) umfasst, wobei der zweite Teil von dem Atemwegen des Patienten getrennt und mit dem Satz von optischen Elementen des ersten Teils durch eine optische Leitung verbunden ist.

10. System nach Anspruch 9, wobei die Infrarotquelle (11, 58) und der Infrarotdetektor (13, 56) in der Nähe des Atemwegs des Patienten positioniert sind, wobei der Gaszusammensetzungssensor (32) ferner ein reflektierendes Element (5, 46, 60) umfasst, das innerhalb eines zwischen der Infrarotquelle (11, 58) und dem Infrarotdetektor (13, 56) definierten optischen Wegs positioniert ist.

11. Verfahren (100) zum Überwachen eines Blutflusses, der nicht mit der beatmeten Lunge eines Patienten interagiert, mit einem System nach einem der Ansprüche 1 bis 10, wobei das Verfahren Folgendes umfasst:

a) Abgeben (110) einer ersten Anzahl von Atemzügen an den Patienten, wobei die Atemzüge der ersten Anzahl von Atemzügen ein Atemgas und eine Menge eines blutlöslichen metabolisch inerten Gases enthalten, wobei jeder Atemzug der ersten Anzahl von Atemzügen eine klinisch akzeptable Konzentration an Sauerstoff enthält;
b) Bestimmen (120) eines endexspiratorischen Konzentrationswerts des blutlöslichen metabolisch inerten Gases in Ausatemzügen des Patienten während der Abgabe der ersten Anzahl von Atemzügen an den Patienten;
c) Wiederholen von b), bis mindestens eines der folgenden eintritt: i) eine vorbestimmte Zeitdauer nach a) verstreicht, oder ii) mindestens zwei aufeinander folgende endexspiratorische Konzentrationswerte des blutlöslichen metabolisch inerten Gases in den Ausatemzügen des Patienten im Wesentlichen gleich sind (140);
d) nach c) Abgeben (150) einer zweite Anzahl von Atemzügen an den Patienten, wobei die Atemzüge der zweiten Anzahl von Atemzügen kein blutlösliches metabolisch inertes Gas enthalten;
e) Bestimmen (160) eines endexspiratorischen Konzentrationswerts des blutlöslichen metabolisch inerten Gases in Ausatemzügen des Patienten während der Abgabe der zweiten Anzahl von Atemzügen an den Patienten; und
f) Schätzen eines relativen Anteils des Blutflusses, der nicht mit der beatmeten Lunge des Patienten interagiert, basierend auf Schwankungen der endexspiratorischen Konzentrationswerte in der ersten Anzahl von Atemzügen oder in der zweiten Anzahl von Atemzügen oder in der ersten und zweiten Anzahl von Atemzügen.

12. Verfahren nach Anspruch 11, wobei das Abgeben (110) der ersten Anzahl von Atemzügen an den Patienten ferner das Steuern (112) eines Gehalts des blutlöslichen metabolisch inerten Gases in jedem weiteren an den Patienten abgegebenen Atemzug im Hinblick auf das Konstanthalten einer Konzentration des blutlöslichen metabolisch inerten Gases in einem Alveolarraum des Patienten umfasst.

13. Verfahren nach Anspruch 11, wobei das Abgeben (110) der ersten Anzahl von Atemzügen an den Patienten ferner das Konstanthalten (114) einer Konzentration des im blutlöslichen metabolisch inerten Gases in jedem an den Patienten abgegebenen Atemzug umfasst.

**Revendications**

1. Système de surveillance d'un flux sanguin n'interagissant pas avec les poumons ventilés d'un patient, comprenant :

une première source (20) d'un gaz respiratoire ;
une seconde source (26) contenant un gaz métaboliquement inerte soluble dans le sang ;
un mélangeur de gaz (28) connecté aux première et seconde sources et adapté pour administrer un mélange du gaz respiratoire et du gaz métaboliquement inerte soluble dans le sang dans les voies respiratoires du patient ;
un capteur de composition gazeuse (32) adapté pour mesurer une concentration du gaz métaboliquement inerte soluble dans le sang présent dans les respirations inhalées et expirées dans les voies respiratoires du patient ; et
une unité de commande (24) fonctionnellement connectée au mélangeur de gaz (28) et au capteur de composition gazeuse (32), dans lequel l'unité de commande (24) est adaptée pour :

a) amener le mélangeur de gaz (28) à administrer un premier nombre de respirations au patient, les respirations du premier nombre de respirations contenant le gaz respiratoire et une quantité de gaz métaboliquement inerte soluble dans le sang, chaque respiration du premier nombre de respirations contenant une concentration d'oxygène cliniquement acceptable ;
b) déterminer, sur la base de mesures provenant du capteur de composition gazeuse (32), une valeur de concentration de fin d'expiration du gaz métaboliquement inerte soluble dans le sang dans les respirations expirées du patient pendant l'administration du premier nombre de respirations au patient ;

c) répéter b) jusqu'à ce qu'au moins l'un des événements suivants se produise : i) une durée prédéterminée après a) s'est écoulée, ou ii) au moins deux valeurs successives de concentration de fin d'expiration du gaz métaboliquement inerte soluble dans le sang dans les respirations expirées du patient sont sensiblement égales ;

d) après c), amener le mélangeur de gaz (28) à administrer un second nombre de respirations au patient, les respirations du second nombre de respirations ne contenant aucun gaz métaboliquement inerte soluble dans le sang ;

e) déterminer, sur la base de mesures provenant du capteur de composition gazeuse (32), une valeur de concentration de fin d'expiration du gaz métaboliquement inerte soluble dans le sang dans les respirations expirées du patient pendant l'administration du second nombre de respirations au patient ; et

f) estimer une fraction relative du flux sanguin qui n'interagit pas avec les poumons ventilés du patient sur la base des variations des valeurs de concentration de fin d'expiration dans le premier nombre de respirations, ou dans le second nombre de respirations ou dans les premier et second nombres de respirations.

2. Système selon la revendication 1, comprenant en outre un circuit de voies respiratoires (22) adapté pour être connecté aux voies respiratoires du patient.

3. Système selon la revendication 1 ou 2, dans lequel la première source (20) du gaz respiratoire est un ventilateur mécanique (20).

4. Système selon l'une quelconque des revendications 1 à 3, comprenant en outre une source d'oxygène (26) fonctionnellement connectée au mélangeur de gaz (28), dans lequel l'unité de commande (24) est en outre adaptée pour amener le mélangeur de gaz (28) à injecter de l'oxygène dans n'importe quelle respiration administrée au patient lorsqu'une proportion d'oxygène dans les respirations administrées au patient et mesurée par le capteur de composition gazeuse (32) est inférieure à un seuil prédéterminé.

5. Système selon l'une quelconque des revendications 1 à 4, dans lequel le gaz métaboliquement inerte soluble dans le sang est l'oxyde nitreux.

6. Système selon l'une quelconque des revendications 1 à 5, dans lequel dans un premier mode de fonctionnement, l'unité de commande (24) fixe le premier nombre de respirations pour que leur durée combinée soit inférieure à un temps moyen de recirculation sanguine dans les poumons du patient.

7. Système selon l'une quelconque des revendications 1 à 6, dans lequel dans un second mode de fonctionnement, l'unité de commande (24) fixe le premier nombre de respirations pour que leur durée dépasse le temps moyen de recirculation sanguine dans les poumons du patient.

8. Système selon la revendication 7, dans lequel l'administration du premier nombre de respirations se poursuit jusqu'à ce que les mesures du capteur de composition gazeuse (32) montrent une valeur stable de concentration de fin d'expiration du gaz métaboliquement inerte soluble dans le sang dans les respirations expirées du patient.

9. Système selon l'une quelconque des revendications 1 à 8, dans lequel le capteur de composition gazeuse (32) comprend :

une première partie comprenant un ensemble d'éléments optiques (4, 5, 6, 10, 44, 46, 48, 52, 60) adaptés pour permettre à la lumière infrarouge d'être projetée sur les voies respiratoires du patient ; et
une seconde partie comprenant une source infrarouge (11, 58) et un détecteur infrarouge (13, 56), la seconde partie étant disjointe des voies respiratoires du patient et étant reliée à l'ensemble d'éléments optiques de la première partie par un conduit optique.

10. Système selon la revendication 9, dans lequel la source infrarouge (11, 58) et le détecteur infrarouge (13, 56) sont positionnés à proximité des voies respiratoires du patient, le capteur de composition gazeuse (32) comprenant en outre un élément réfléchissant (5, 46, 60) positionné à l'intérieur d'un trajet optique défini entre la source infrarouge (11, 58) et le détecteur infrarouge (13, 56).

11. Procédé (100) de surveillance d'un flux sanguin qui n'interagit pas avec les poumons ventilés d'un patient avec un système selon l'une quelconque des revendications 1 à 10, dans lequel le procédé comprend :

a) l'administration (110) d'un premier nombre de respirations au patient, les respirations du premier nombre de respirations contenant un gaz respiratoire et une quantité d'un gaz métaboliquement inerte soluble dans le sang, chaque respiration du premier nombre de respirations contenant une concentration d'oxygène cliniquement acceptable ;

b) la détermination (120) d'une valeur de concentration de fin d'expiration du gaz métaboliquement inerte soluble dans le sang dans les respirations expirées du patient pendant l'administration du premier nombre de respirations au patient ;

c) la répétition de b) jusqu'à ce qu'au moins l'un des événements suivants se produise : *i)* une durée prédéterminée après a) s'est écoulée, ou *ii)* au moins deux valeurs successives de concentration de fin d'expiration du gaz métaboliquement inerte soluble dans le sang dans les respirations expirées du patient sont sensiblement égales (140) ;

d) après c), l'administration (150) d'un second nombre de respirations au patient, les respirations du second nombre de respirations ne contenant aucun gaz métaboliquement inerte soluble dans le sang ;

e) la détermination (160) d'une valeur de concentration de fin d'expiration du gaz métaboliquement inerte soluble dans le sang dans les respirations expirées du patient pendant l'administration du second nombre de respirations au patient ; et

f) l'estimation d'une fraction relative du flux sanguin qui n'interagit pas avec les poumons ventilés du patient sur la base des variations des valeurs de concentration de fin d'expiration dans le premier nombre de respirations, ou dans le second nombre de respirations ou dans les premier et second nombres de respirations.

12. Procédé selon la revendication 11, dans lequel l'administration (110) du premier nombre de respirations au patient comprend en outre le contrôle (112) d'une teneur en gaz métaboliquement inerte soluble dans le sang dans chaque respiration successive administrée au patient en vue de maintenir constante une concentration du gaz métaboliquement inerte soluble dans le sang dans un espace alvéolaire du patient.

13. Procédé selon la revendication 11, dans lequel l'administration (110) du premier nombre de respirations au patient comprend en outre le maintien constante (114) d'une concentration du gaz métaboliquement inerte soluble dans le sang dans chaque respiration administrée au patient.

Figure 1

Figure 2a

Figure 2b

Figure 3

Figure 4b

Figure 4a

Figure 5a

Figure 5b

Figure 6b

Figure 6a

Figure 6c

EP 3 833 254 B1

Figure 7

**EP 3 833 254 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6402697 B1
- CA 2018050957
- CA 2018050957 W